# EUROPÄISCHE PATENTSCHRIFT

(11) **EP 2 415 417 B1**
(45) Veröffentlichungstag und Bekanntmachung des Hinweises auf die Patenterteilung: **05.04.2017**
(21) Anmeldenummer: 11174482.7
(22) Anmeldetag: 19.07.2011
(51) Int. Cl.: A61B 6/04, A61B 6/00, A61B 34/30, A61B 34/37, A61B 90/00

(54) **Medizinischer Arbeitsplatz**
Medical workstation
Poste de travail médical

(30) Priorität: 02.08.2010 DE 102010038800
(43) Veröffentlichungstag der Anmeldung: 08.02.2012
(73) Patentinhaber: KUKA Roboter GmbH, 86165 Augsburg (DE)
(72) Erfinder: Müller, Michael, 86150 Augsburg (DE)
(74) Vertreter: Patentanwälte Funk & Böss GbR

(56) Entgegenhaltungen:
- WO-A2-2006/069288
- DE-A1-102008 019 345
- DE-A1-102008 022 924
- US-A1- 2008 119 714
- US-A1- 2009 024 025

## Beschreibung

Die Erfindung betrifft einen medizinischen Arbeitsplatz, der ein medizintechnisches Gerät und eine verstellbare Patientenlagerungsvorrichtung umfasst. Das medizintechnische Gerät und die Patientenlagerungsvorrichtung umfassen jeweils wenigstens einen Roboter.

Roboter sind Arbeitsmaschinen, die zur automatischen Handhabung und/ oder Bearbeitung von Objekten mit Werkzeugen ausgerüstet werden können und in mehreren Bewegungsachsen beispielsweise hinsichtlich Orientierung, Position und Arbeitsablauf programmierbar sind. Roboter weisen üblicherweise einen Roboterarm mit mehreren über Gelenke verbundene Glieder und programmierbare Steuerungen (Steuervorrichtungen) auf, die während des Betriebs die Bewegungsabläufe des Roboters steuern bzw. regeln. Die Glieder werden über Antriebe, die von der Steuervorrichtung angesteuert werden, insbesondere bezüglich der Bewegungsachsen bewegt.

Die DE 10 2008 019 345 A1 offenbart einen medizinischen Arbeitsplatz mit einem Röntgengerät und einer Patientenliege. Das Röntgengerät weist einen Roboter auf, an dessen Roboterarm ein C-Bogen mit einer Röntgenstrahlenquelle und einem Röntgenstrahlempfänger befestigt ist. Die Patientenliege ist an einem Roboterarm eines weiteren Roboters befestigt.

Die DE 10 2008 016 414 A1 offenbart einen medizinischen Arbeitsplatz mit einer Patientenliege und einer Röntgenvorrichtung. Die Röntgenstrahlenquelle der Röntgenvorrichtung ist an einem Roboterarm eines Roboters und der Röntgenstrahlenempfänger ist an einem Roboterarm eines weiteren Roboters befestigt. Die Steuervorrichtungen der beiden Roboter sind miteinander gekoppelt und als ein Master-Slave-System ausgebildet, bei dem einer der Steuervorrichtungen als der Master und die andere Steuervorrichtung als der Slave ausgebildet ist. Die als Master ausgebildete Steuervorrichtung steuert die als Slave ausgebildete Steuervorrichtung derart an, dass die Röntgenstrahlenquelle und der Röntgenstrahlenempfänger stets zueinander in einem vorab festgelegten Abstand ausgerichtet sind.

WO2006/069288 offenbart einen Arbeitsplatz mit einem chirurgischen Roboter und einer Patientenliege, wobei der chirurgische Roboter in Abhängigkeit zu der Liege bewegt wird.

Aufgabe der Erfindung ist es, einen verbesserten medizinischen Arbeitsplatz mit einem medizintechnischen Gerät und einer Patientenlagerungsvorrichtung anzugeben, wobei das medizintechnische Gerät und die Patientenlagerungsvorrichtung jeweils wenigstens einen Roboter aufweisen.

Die Aufgabe der Erfindung wird gelöst durch einen medizinischen Arbeitsplatz, aufweisend
- ein medizintechnisches Gerät, welches eine medizintechnische Einrichtung und wenigstens einen ersten Roboter aufweist, der einen mehrere Glieder aufweisenden ersten Roboterarm und einen eine Bewegung des ersten Roboterarms steuernde erste Steuervorrichtung aufweist, wobei an einer ersten Befestigungsvorrichtung des ersten Roboterarms die medizintechnische Einrichtung befestigt ist, und
- eine Patientenlagerungsvorrichtung, die eine Patientenliege und einen zweiten Roboter aufweist, der einen mehrere Glieder aufweisenden zweiten Roboterarm und einen eine Bewegung des zweiten Roboterarms steuernde zweite Steuervorrichtung aufweist, wobei an einer zweiten Befestigungsvorrichtung des zweiten Roboterarms die Patientenliege befestigt ist,
wobei die beiden Steuervorrichtungen miteinander gekoppelt und als ein Master-Slave-System ausgebildet sind, bei dem eine der Steuervorrichtungen als der Master und die andere Steuervorrichtung als der Slave ausgebildet ist, die als Master ausgebildete Steuervorrichtung die als Slave ausgebildete Steuervorrichtung derart ansteuert, dass bei einer ersten Bewegung der Befestigungsvorrichtung des Roboters, dessen Steuervorrichtung als der Master ausgebildet ist, die als Slave ausgebildete Steuervorrichtung ihren Roboterarm derart bewegt, sodass die Befestigungsvorrichtung des Roboters, dessen Steuervorrichtung als Slave ausgebildet ist, eine zweite Bewegung ausführt, aufgrund derer die Patientenliege und die medizintechnische Einrichtung stets relativ zueinander gleichbleibend ausgerichtet sind.

Der erfindungsgemäße medizinische Arbeitsplatz weist demnach wenigstens zwei Roboter auf, die jeweils mehrere Achsen und jeweils eine Befestigungsvorrichtung, insbesondere einen Flansch, umfassen. Einer der Roboter ist Teil der Patientenlagerungsvorrichtung, auf deren Patientenliege ein Lebewesen z.B. während einer Behandlung mit dem medizintechnischen Gerät liegen kann. Der andere Roboter bzw. die anderen Roboter sind Teil des medizintechnischen Gerätes.

Die medizintechnische Einrichtung ist z.B. als eine bildgebende medizintechnische Einrichtung ausgebildet. Ein Beispiel einer bildgebenden medizintechnischen Einrichtung ist eine Röntgenvorrichtung.

Jeder der Roboter umfasst seine eigene Steuervorrichtung. Die jeweiligen Steuervorrichtungen steuern im Betrieb des erfindungsgemäßen medizinischen Arbeitsplatzes ihre jeweiligen Roboterarme. Dazu können, wie dies dem Fachmann allgemein bekannt ist, die Roboter mit elektrischen Antrieben versehen sein, die wiederum von den relevanten Steuervorrichtungen angesteuert werden. Somit umfasst der erfindungsgemäße medizinische Arbeitsplatz keine zentrale Steuervorrichtung, die alle Roboterarme der Roboter zusammen ansteuert. Dadurch ist es möglich, zwei oder mehrere Standardroboter mit jeweils einer für sie bestimmten Steuervorrichtung zu verwenden. Dies kann eine flexiblere Ausführung des erfindungsgemäßen medizinischen Arbeitsplatzes zur Folge haben.

Erfindungsgemäß soll die Patientenliege zumindest in einem Betriebsmodus des medizinischen Arbeitsplatzes stets relativ zueinander gleichbleibend ausgerichtet sein. Um dies zu erreichen, sind die Steuervorrichtungen miteinander gekoppelt und als Master-Slave-System ausgebildet. Eine der Steuervorrichtung ist dabei als Master und die andere Steuervorrichtung als Slave ausgebildet.

Der erfindungsgemäße medizinische Arbeitsplatz kann derart eingerichtet sein, dass die Patientenliege stets mittig bezüglich des Rötengenstrahlenempfängers und der Röntgenstrahlenquelle angeordnet ist, d.h. dass die Patientenliege jeweils denselben Abstand zum Röntgenstrahlenempfänger und zur Röntgenstrahlenquelle aufweist. Es ist aber auch möglich, dass sich der Abstand zwischen der Patientenliegen und dem Röntgenstrahlenempfänger und der Abstand zwischen der Patientenliege und der Röntgenstrahlensender unterscheiden, jedoch stets gleich bleiben. Die beiden Abstände können z.B. ein Verhältnis von 1:2, 2:3, 1:4 aufweisen.

Im Betrieb des erfindungsgemäßen medizinischen Arbeitsplatzes ist es demnach möglich, dass die als Master ausgebildete Steuervorrichtung während der ersten Bewegung der Befestigungsvorrichtung ihres Roboters die als Slave ausgebildete Steuervorrichtung ansteuert, sodass diese wiederum ihren Roboterarm derart ansteuert, dass die relevante Befestigungsvorrichtung der ersten Bewegung der anderen Befestigungsvorrichtung derart folgt, dass die medizintechnische Einrichtung und die Patientenliege stets relativ zueinander definiert, insbesondere gleichbleibend, ausgerichtet sind.

Dabei kann es vorgesehen sein, dass entweder die erste oder die zweite Steuervorrichtung als Master ausgebildet ist. Ist z.B. die erste Steuervorrichtung als Master ausgebildet, dann folgt die Patientenliege automatisch einer Bewegung der medizintechnischen Einrichtung. Ist die zweite Steuervorrichtung als Master ausgebildet, folgt automatisch die medizintechnische Einrichtung einer Bewegung der Patientenliege.

Nach einer Ausführungsform des erfindungsgemäßen medizinischen Arbeitsplatzes steuert die als Master ausgebildete Steuervorrichtung ihren Roboterarm derart automatisch an, dass die relevante Befestigungsvorrichtung die erste Bewegung durchführt. Gemäß dieser Variante ergibt sich eine vollautomatische Bewegung des gesamten medizinischen Arbeitsplatzes.

Es ist auch möglich, dass die erste Bewegung manuell durchgeführt wird. Dies erfolgt nach einer Ausführungsform des erfindungsgemäßen medizinischen Arbeitsplatzes durch ein manuellen Führens zumindest eines Teils der medizintechnischen Einrichtung oder aufgrund eines manuellen Führens der Patientenliege bzw. des relevanten Roboterarms.

Gemäß einer weiteren Ausführungsform des erfindungsgemäßen medizinischen Arbeitsplatzes weist dieser mit der als Master ausgebildeten Steuervorrichtung gekoppelte Eingabemittel auf, mittels deren die als Master ausgebildete Steuervorrichtung ihren Roboterarm derart ansteuert, dass die relevante Befestigungsvorrichtung die erste Bewegung aufgrund einer manuellen Eingabe in die Eingabemittel durchführt. Die manuellen Eingabemittel sind z.B. ein Bedienhandgerät.

Nach einer weiteren Ausführungsform des medizinischen Arbeitsplatzes übermittelt die als Master ausgebildete Steuervorrichtung der als Slave ausgebildeten Steuervorrichtung während der ersten Bewegung eine Information über die aktuelle Position und Orientierung ihrer Befestigungsvorrichtung. Daraufhin kann die als Slave ausgebildete Steuervorrichtung aufgrund der relativen Lage des Roboterarm, dessen Steuervorrichtung als Master ausgebildet ist, relativ zum Roboterarm, dessen Steuervorrichtung als Slave ausgebildet ist, und der Information über die aktuelle Position und Orientierung der Befestigungsvorrichtung des Roboters, dessen Steuervorrichtung als Master ausgebildet ist, den Roboterarm des Roboters, dessen Steuervorrichtung als Slave ausgebildet ist, derart bewegen, sodass die Befestigungsvorrichtung dieses Roboters eine Position und Orientierung aufweist, in der die Patientenliege und die medizintechnische Einrichtung stets relativ zueinander gleichbleibend ausgerichtet sind.

Ist die Steuervorrichtung des Roboters, der Teil des medizinischen Gerätes ist, als Master ausgebildet, dann kann es auch vorgesehen sein, dass diese Steuervorrichtung eine Information über die aktuelle Position und Orientierung der an ihrer Befestigungsvorrichtung angeordneten medizintechnische Einrichtung übermittelt.

Ist die Steuervorrichtung des Roboters, der Teil der Patientenlagerungsvorrichtung, als Master ausgebildet, dann kann es auch vorgesehen sein, dass diese Steuervorrichtung eine Information über die aktuelle Position und Orientierung der an ihrer Befestigungsvorrichtung angeordneten Patientenliege übermittelt.

Der erfindungsgemäße medizinische Arbeitsplatz erlaubt je nach Ausführungsform einen Abgleich zwischen den Positionen des Patientenpositionierers bzw. der Patientenlagerungsvorrichtung und wenigstens einem weiteren medizintechnischen Gerät ohne zusätzliche externe Steuerung zu realisieren. Dadurch kann eine zeitliche Verzögerung zumindest teilweise, wenn nicht gar weitestgehend eliminiert und die Schwankungen in der Entfernung zwischen dem Patientenpositionierer bzw. der Patientenlagerungsvorrichtung und dem medizintechnischen Gerät zumindest verkleinert, wenn nicht gar minimiert oder vermieden werden. Der Aufwand für eine Kollisionsberechnung kann relativ gering sein.

Für den erfindungsgemäßen medizinischen Arbeitplatz wird je nach Ausführungsform ein roboterbasierter Patientenpositionierer bzw. eine roboterbasierte Patientenlagerungsvorrichtung eingesetzt und zusätzlich wenigstens ein medizintechnisches Gerät ebenfalls per Roboter geführt. Die beiden roboterbasierten Systeme können insbesondere miteinander synchron positioniert werden. Eine relative Ausrichtung ist auch möglich, die stets klar definiert sein kann.

Gegebenfalls durch den Einsatz einer Kollisions-Vermeidung können Kollisionen zwischen dem Patientenpositionierer bzw. der Patientenlagervorrichtung und dem medizintechnischen Gerät vermieden werden.

Ausführungsbeispiele der Erfindung sind exemplarisch in den beigefügten schematischen Zeichnungen dargestellt. Es zeigen:
- Fig. 1: einen medizinischen Arbeitsplatz und
- Fig. 2: einen weiteren medizinischen Arbeitsplatz.

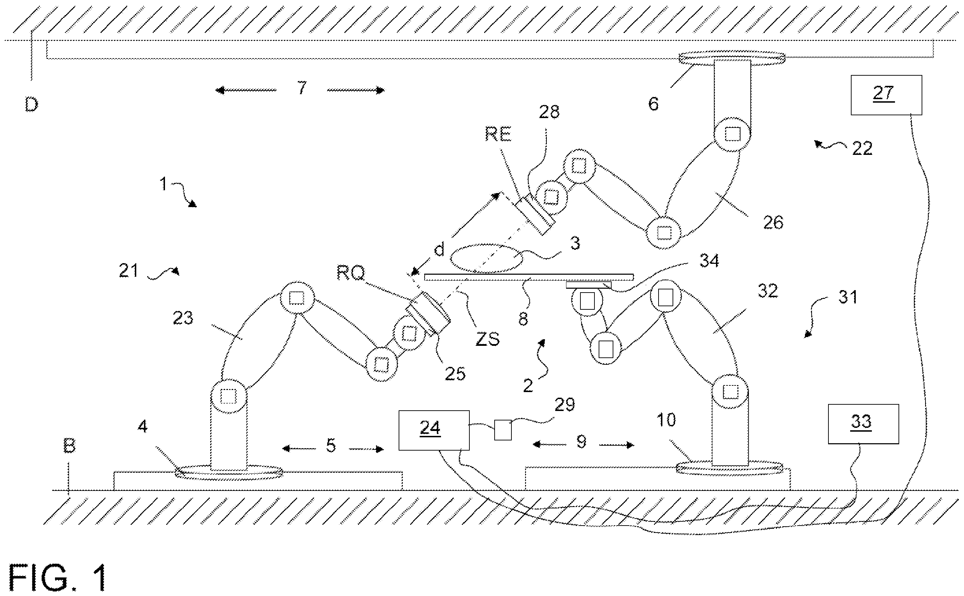

Die Fig. 1 zeigt einen medizinischen Arbeitsplatz mit einer Röntgenvorrichtung 1 und einer verstellbaren Patientenlagerungsvorrichtung 2 zum Lagern eines Lebewesens 3. Die Röntgenvorrichtung 1 ist ein Beispiel eines medizintechnischen Gerätes und insbesondere ein Beispiel eines bildgebenden medizintechnischen Gerätes.

Die in der Fig. 1 gezeigte Röntgenvorrichtung 1 weist einen ersten Roboter 21 und einen zweiten Roboter 22 auf, sowie eine Röntgenstrahlenquelle RQ und einen Röntgenstrahlenempfänger RE.

Der erste Roboter 21 weist einen Roboterarm 23 und eine Steuervorrichtung 24 auf. Der Roboterarm 23 umfasst mehrere Glieder, die Glieder verbindende Gelenke, mit der Steuervorrichtung 24 in nicht dargestellter Weise verbundene Antriebe, insbesondere elektrische Antriebe zum Bewegen der Glieder, und eine Befestigungsvorrichtung 25 z.B. in Form eines Flansches. Auf der Steuervorrichtung 24 läuft ein Rechnerprogramm, sodass die Steuervorrichtung 24 die Antriebe derart ansteuert, dass die Position und Orientierung der Befestigungsvorrichtung 25 im Wesentlichen frei im Raum ausgerichtet werden kann. Die elektrischen Antriebe des ersten Roboters 21 umfassen beispielsweise jeweils einen elektrischen Motor und gegebenenfalls eine die Motoren ansteuernde Leistungselektronik. An der Befestigungsvorrichtung 25 des Roboterarms 23 des ersten Roboters 21 ist die Röntgenstrahlenquelle RQ befestigt.

Der zweite Roboter 22 weist einen Roboterarm 26 und eine Steuervorrichtung 27 auf. Der Roboterarm 26 umfasst mehrere Glieder, die Glieder verbindende Gelenke, mit der Steuervorrichtung 27 in nicht dargestellter Weise verbundene Antriebe, insbesondere elektrische Antriebe zum Bewegen der Glieder, und eine Befestigungsvorrichtung 28 z.B. in Form eines Flansches. Auf der Steuervorrichtung 27 läuft ein Rechnerprogramm, sodass die Steuervorrichtung 27 die Antriebe derart ansteuert, dass die Position und Orientierung der Befestigungsvorrichtung 28 im Wesentlichen frei im Raum ausgerichtet werden kann. Die elektrischen Antriebe des zweiten Roboters 22 umfassen beispielsweise jeweils einen elektrischen Motor und gegebenenfalls eine die Motoren ansteuernde Leistungselektronik. An der Befestigungsvorrichtung 28 des Roboterarms 26 des zweiten Roboters 22 ist der Röntgenstrahlenempfänger RE befestigt.

Im Falle des vorliegenden Ausführungsbeispiels umfasst die Patientenlagerungsvorrichtung 2 eine Patientenliege 8 und einen dritten Roboter 31. Auf der Patientenliege 8 liegt das Lebewesen 3.

Der dritte Roboter 31 weist einen Roboterarm 32 und eine Steuervorrichtung 33 auf. Der Roboterarm 32 umfasst mehrere Glieder, die Glieder verbindende Gelenke, mit der Steuervorrichtung 33 in nicht dargestellter Weise verbundene Antriebe, insbesondere elektrische Antriebe zum Bewegen der Glieder, und eine Befestigungsvorrichtung 34 z.B. in Form eines Flansches. Auf der Steuervorrichtung 33 läuft ein Rechnerprogramm, sodass die Steuervorrichtung 33 die Antriebe derart ansteuert, dass die Position und Orientierung der Befestigungsvorrichtung 34 im Wesentlichen frei im Raum ausgerichtet werden kann. Die elektrischen Antriebe des dritten Roboters 31 umfassen beispielsweise jeweils einen elektrischen Motor und gegebenenfalls eine die Motoren ansteuernde Leistungselektronik. An der Befestigungsvorrichtung 34 des Roboterarms 32 des dritten Roboters 31 ist die Patientenliege 8 befestigt.

Im Falle des vorliegenden Ausführungsbeispiels ist der erste Roboter 21 bzw. dessen Roboterarm 23 an einer am Boden B des medizinischen Arbeitsplatzes befestigten und insbesondere schienengebundenen Lineareinheit 4 befestigt, mittels derer der erste Roboter 21 bzw. dessen Roboterarm 23 entlang eines Doppelpfeils 5 bewegbar ist. Der zweite Roboter 22 bzw. dessen Roboterarm 26 ist an einer an der Decke D des medizinischen Arbeitsplatzes befestigten und insbesondere schienengebundenen Lineareinheit 6 befestigt, mittels derer der zweite Roboter 22 bzw. dessen Roboterarm 26 entlang eines Doppelpfeils 7 bewegbar ist. Der dritte Roboter 31 bzw. dessen Roboterarm 32 ist an einer am Boden B des medizinischen Arbeitsplatzes befestigten und insbesondere schienengebundenen Lineareinheit 10 befestigt, mittels derer der dritte Roboter 31 bzw. dessen Roboterarm 32 entlang eines Doppelpfeils 9 bewegbar ist.

Die Lineareinheiten 4, 6, 10 umfassen jeweils in den Figuren nicht dargestellte Antriebe, wobei der Antrieb der dem ersten Roboter 21 zugeordneten Lineareinheit 4 mit der Steuervorrichtung 24 des ersten Roboters 21, der Antrieb der dem zweiten Roboter 22 zugeordneten Lineareinheit 6 mit der Steuervorrichtung 27 des zweiten Roboters 22 und der Antrieb der dem dritten Roboter 31 zugeordneten Lineareinheit 10 mit der Steuervorrichtung 33 des dritten Roboters 31 verbunden ist.

Im Betrieb der Roboter 21, 22, 31 steuert die Steuervorrichtung 24 des ersten Roboters 21 die dem ersten Roboter 21 zugeordnet Lineareinheit 4, um den Roboterarm 23 des ersten Roboters 21 längs des Doppelpfeils 5 zu bewegen, die Steuervorrichtung 27 des zweiten Roboters 22 steuert die dem zweiten Roboter 22 zugeordnet Lineareinheit 6, um den Roboterarm 26 des zweiten Roboters 22 längs des Doppelpfeils 7 zu bewegen, und die Steuervorrichtung 33 des dritten Roboters 31 die dem dritten Roboter 31 zugeordnet Lineareinheit 10, um den Roboterarm 32 des dritten Roboters 32 längs des Doppelpfeils 9 zu bewegen.

Im Falle des vorliegenden Ausführungsbeispiels kann in einem ersten Betriebsmodus z.B. ein in den Figuren nicht näher dargestellter Arzt den ersten Roboter 21 mittels eines mit dessen Steuervorrichtung 24 verbundenen Bediengeräts 29 derart bedienen, dass die Steuervorrichtung 24 die Antriebe des ersten Roboters 21 derart ansteuert, dass die Befestigungsvorrichtung 25 des Roboterarms 23 des ersten Roboters 21 und somit die Röntgenstrahlenquelle RQ eine vom Arzt bestimmte Bewegung durchführt. Somit ist es dem Arzt möglich, die Röntgenstrahlenquelle RQ relativ zum Lebewesen 3 in gewünschter Weise auszurichten, um eine Röntgenaufnahme von einem Körperbereich des Lebewesens 3 herzustellen.

Für die Röntgenaufnahme, die der Arzt z.B. mittels eines nicht näher dargestellten Eingabemittels des Bediengerätes 29 auslösen kann, erzeugt die Röntgenstrahlenquelle RQ eine Röntgenstrahlung mit einem Zentralstrahl ZS.

Für die Aufnahme wird die Röntgenstrahlung beim Durchtritt durch das Lebewesen 3 teilweise geschwächt und trifft auf den Röntgenstrahlenempfänger RE auf. Dieser wandelt die auftreffende Röntgenstrahlung in ein der Röntgenstrahlung entsprechendes elektrisches Signal um, dem wiederum ein nicht näher dargestelltes Röntgenbild vom relevanten Körperbereich des Lebewesens 3 zugeordnet ist. Das Röntgenbild kann z.B. mittels eines der Übersicht halber nicht näher dargestellten Bildschirms betrachtet werden.

Damit das Röntgenbild eine zumindest zufrieden stellende Qualität aufweist, sollte während der Röntgenaufnahme der Röntgenstrahlenempfänger RE relativ zur Röntgenstrahlenquelle RQ in einem vorab festgelegten Abstand d ausgerichtet sein. Im Falle des vorliegenden Ausführungsbeispiels kann es noch vorgesehen sein, dass die Patientenliege 8 stets relativ zur Röntgenstrahlenquelle RQ bzw. zum Röntgenstrahlenempfänger RE definiert, insbesondere gleichbleibend, ausgerichtet ist. Dies wird im Falle des vorliegenden Ausführungsbeispiels folgendermaßen realisiert:
Die Steuervorrichtungen 24, 27, 33 der drei Roboter 21, 22, 31 sind als ein Master-Slave System ausgebildet, wobei im Falle des vorliegenden Ausführungsbeispiels die Steuervorrichtung 24 des ersten Roboters 21 als der Master und die Steuervorrichtungen 27, 33 des zweiten Roboters 22 bzw. des dritten Roboters 31 als Slave ausgebildet sind. Aufgrund der Eingabe in das Bediengerät 29 steuert die Steuervorrichtung 24 die elektrischen Antriebe des ersten Roboters 21 und gegebenenfalls der Lineareinheit 4 derart an, dass die Befestigungsvorrichtung 26 des ersten Roboters 21 und somit die Röntgenstrahlenquelle RQ die Bewegung ausführen.

Gleichzeitig übermittelt die Steuervorrichtung 24 des ersten Roboters 21 der Steuervorrichtung 27 des zweiten Roboters 22 und der Steuervorrichtung 33 des dritten Roboters 31 eine Information über die aktuelle Position und Orientierung seiner Befestigungsvorrichtung 25, die im Falle des vorliegenden Ausführungsbeispiels eine Information über die Position und Orientierung eines dieser Befestigungsvorrichtung 25 zugeordneten Koordinatensystems darstellt. Den Steuervorrichtungen 27, 33 des zweiten und dritten Roboters 22, 31 stehen außerdem eine Information über die relative Lage (Orientierung und Position) zwischen dem Koordinatensystem der Befestigungsvorrichtung 25 des ersten Roboters 21 und gegebenenfalls einem Koordinatensystem der Röntgenstrahlenquelle RQ und einem Koordinatensystem der Patientenliege 8 zur Verfügung.

Ferner wurden die Roboterarme 23, 26, 32 der drei Roboter 21, 22, 31 vorab vermessen, sodass der Steuervorrichtung 27 des zweiten Roboters 22 auch eine Information über die relative Lage der Roboterarme 23, 26 des ersten und des zweiten Roboters 21, 22 und der Steuervorrichtung 33 des dritten Roboters 31 auch eine Information über die relative Lage der Roboterarme 23, 32 des ersten und des dritten Roboters 21, 31 zueinander bekannt ist. Eine Information über die aktuelle Position des mittels der Lineareinheit 4 verfahrbaren Roboterarms 23 des ersten Roboters 21 übermittelt die Steuervorrichtung 24 des ersten Roboters 21 ebenfalls den Steuervorrichtungen 27, 33 des zweiten und dritten Roboters 22, 31 während der Bewegung der Befestigungsvorrichtung 25 des ersten Roboters 21.

Somit ist es der Steuervorrichtung 27 des zweiten Roboters 22 möglich, die aktuelle Position und Orientierung der Befestigungsvorrichtung 25 des ersten Roboters 21 bzw. der Röntgenstrahlenquelle RQ zu errechnen, um wiederum mittels eines auf der Steuervorrichtung 27 des zweiten Roboters 22 laufenden Rechnerprogramms die Antriebe des zweiten Roboters 22 derart anzusteuern, dass die Befestigungsvorrichtung 28 des zweiten Roboters 22 und insbesondere ein Tool Center Point des Röntgenstrahlenempfängers RQ derart ausgerichtet wird, dass dieser den vorab festgelegten Abstand d zur Röntgenstrahlenquelle RQ aufweist und ebenfalls auf diese ausgerichtet ist.

Die oben beschriebenen Informationen übermittelt die Steuervorrichtung 24 des ersten Roboters 21 kontinuierlich der Steuervorrichtung 27 des zweiten Roboters 22 während der Bewegung, sodass die Steuervorrichtung 27 des zweiten Roboters 22 stets die Antriebe des zweiten Roboters 22 derart ansteuern kann, damit der Röntgenstrahlenempfänger RE stets auf die Röntgenstrahlenquelle RQ im Abstand d ausgerichtet ist.

Der Steuervorrichtung 33 des dritten Roboters 31 ist es ebenfalls möglich, die aktuelle Position und Orientierung der Befestigungsvorrichtung 25 des ersten Roboters 21 bzw. der Röntgenstrahlenquelle RQ zu errechnen, um wiederum mittels eines auf der Steuervorrichtung 33 des dritten Roboters 31 laufenden Rechnerprogramms die Antriebe des dritten Roboters 31 derart anzusteuern, dass die Befestigungsvorrichtung 34 des dritten Roboters 31 und insbesondere ein Tool Center Point der Patientenliege 8 derart ausgerichtet wird, dass diese stets relativ zur Röntgenstrahlenquelle RQ bzw. zum Röntgenstrahlenempfänger RE gleichbleibend in vorgegebenen und errechneten Abstand zur Röntgenstrahlenquelle RQ bzw. zum Röntgenstrahlenempfänger RE ausgerichtet ist.

Die oben beschriebenen Informationen übermittelt die Steuervorrichtung 24 des ersten Roboters 21 kontinuierlich der Steuervorrichtung 33 des dritten Roboters 31 während der Bewegung, sodass die Steuervorrichtung 33 des dritten Roboters 31 stets die Antriebe des dritten Roboters 31 derart ansteuern kann, dass insbesondere gemäß einem Ausführungsbeispiel die Patientenliege 8 stets relativ zur Röntgenstrahlenquelle RQ bzw. zum Röntgenstrahlenempfänger RE derart gleichbleibend ausgerichtet ist, dass das Lebewesen 3 stets zur Hälfte des Abstandes d, d.h. mittig zwischen der Röntgenstrahlenquelle RQ und der Röntgenstrahlenempfänger RE, positioniert ist. Es ist aber auch möglich, dass sich der Abstand zwischen der Patientenliegen 8 und dem Röntgenstrahlenempfänger RE und der Abstand zwischen der Patientenliege 8 und der Röntgenstrahlensender RS unterscheiden, jedoch insbesondere stets gleich bleiben. Die beiden Abstände können z.B. ein Verhältnis von 1:2, 2:3, 1:4 aufweisen.

Im Falle des vorliegenden Ausführungsbeispiels kann die Röntgenvorrichtung 1 in einem zweiten Betriebsmodus betrieben werden. Im zweiten Betriebsmodus bewegt z.B. der Arzt die Röntgenstrahlenquelle RQ manuell z.B. durch Führen oder Ziehen am ersten Roboter 21 oder an der Röntgenstrahlenquelle RQ. Während des manuellen Bewegens übermittelt die Steuervorrichtung 24 des ersten Roboters 21 kontinuierlich eine Information über die Position und Orientierung des Koordinatensystems der Befestigungsvorrichtung 25 des ersten Roboters 21 sowie die Position des ersten Roboters 21 bezüglich der Lineareinheit 4.

Aufgrund dieser Information ist den Steuervorrichtungen 27, 33 der beiden anderen Roboter 22, 31 die Lage der Röntgenstrahlenquelle RQ während der manuellen Bewegung des ersten Roboters 21 stets bekannt. Demnach kann die Steuervorrichtung 27 des zweiten Roboters 22 die Antriebe des zweiten Roboters 22 derart ansteuern, sodass der Tool Center Point des Röntgenstrahlenempfängers RE im Abstand d derart zur Röntgenstrahlenquelle RQ stets ausgerichtet ist, dass die Röntgenstrahlenquelle RQ und der Röntgenstrahlenempfänger RE im Abstand d zueinander ausgerichtet sind. Die Steuervorrichtung 33 des dritten Roboters 31 kann während der Bewegung stets die Antriebe des dritten Roboters 31 derart ansteuern kann, dass die Patientenliege 8 stets relativ zur Röntgenstrahlenquelle RQ bzw. zum Röntgenstrahlenempfänger RE gleichbleibend, d.h. insbesondere mittig zwischen der Röntgenstrahlenquelle RQ und der Röntgenstrahlenempfänger RE, ausgerichtet ist.

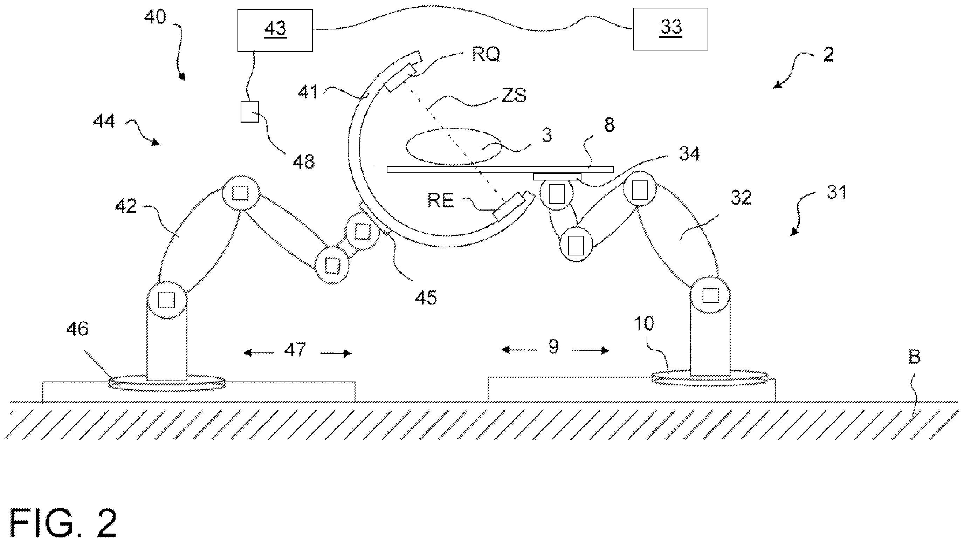

Die Fig. 2 zeigt einen weiteren medizinischen Arbeitsplatz mit einer weiteren Röntgenvorrichtung 40 und der verstellbaren Patientenlagerungsvorrichtung 2 zum Lagern des Lebewesens 3. Die Röntgenvorrichtung 40 ist ein weiteres Beispiel eines medizintechnischen Gerätes und insbesondere ein weiteres Beispiel eines bildgebenden medizintechnischen Gerätes.

Die beiden medizinischen Arbeitsplätze unterscheiden sich im Wesentlichen durch ihre Röntgenvorrichtungen 1, 40. Die in der Fig. 2 gezeigte Röntgenvorrichtung 40 umfasst eine als C-Bogen ausgeführte Trägervorrichtung 41, an deren Enden gegenüberliegend der Röntgenstrahlenempfänger RE und die Röntgenstrahlenquelle RQ befestigt sind, sodass der Zentralstrahl ZS der von der Röntgenstrahlenquelle RQ erzeugten Röntgenstrahlung auf den Röntgenstrahlenempfänger RE treffen kann.

Die Röntgenvorrichtung 40 umfasst einen vierten Roboter 44, der einen Roboterarm 42 und eine Steuervorrichtung 43 aufweist. Der Roboterarm 42 umfasst mehrere Glieder, die Glieder verbindende Gelenke, mit der Steuervorrichtung 43 in nicht dargestellter Weise verbundene Antriebe, insbesondere elektrische Antriebe zum Bewegen der Glieder, und eine Befestigungsvorrichtung 45 z.B. in Form eines Flansches. Auf der Steuervorrichtung 43 läuft ein Rechnerprogramm, sodass die Steuervorrichtung 43 die Antriebe derart ansteuert, dass die Position und Orientierung der Befestigungsvorrichtung 45 im Wesentlichen frei im Raum ausgerichtet werden kann. Die elektrischen Antriebe des vierten Roboters 44 umfassen beispielsweise jeweils einen elektrischen Motor und gegebenenfalls eine die Motoren ansteuernde Leistungselektronik. An der Befestigungsvorrichtung 45 des Roboterarms 42 des vierten Roboters 44 ist die Trägevorrichtung 41 befestigt.

Im Falle des vorliegenden Ausführungsbeispiels ist der vierte Roboter 44 bzw. dessen Roboterarm 42 an einer am Boden B des medizinischen Arbeitsplatzes befestigten und insbesondere schienengebundenen Lineareinheit 46 befestigt, mittels derer der vierte Roboter 44 bzw. dessen Roboterarm 42 entlang eines Doppelpfeils 47 bewegbar ist.

Die dem vierten Roboter 44 zugeordnete Lineareinheit 46 umfasst einen in den Figuren nicht dargestellten Antrieb, der mit der Steuervorrichtung 43 des vierten Roboters 44 verbunden ist. Im Betrieb des vierten Roboters 44 steuert die Steuervorrichtung 43 des vierten Roboters 44 die dem vierten Roboter 44 zugeordnet Lineareinheit 46, um den Roboterarm 42 des vierten Roboters 44 längs des Doppelpfeils 47 zu bewegen.

Im Falle des vorliegenden Ausführungsbeispiels ist es vorgesehen, dass die Patientenliege 8 in einem Betriebsmodus stets relativ zur Röntgenstrahlenquelle RQ bzw. zum Röntgenstrahlenempfänger RE bzw. zur Trägervorrichtung 41 gleichbleibend ausgerichtet ist. Dies wird im Falle des vorliegenden Ausführungsbeispiels folgendermaßen realisiert:
Die Steuervorrichtungen 33, 43 des dritten und vierten Roboters 31, 44 sind als ein Master-Slave System ausgebildet, wobei im Falle des vorliegenden Ausführungsbeispiels die Steuervorrichtung 43 des vierten Roboters 44 als der Master und die Steuervorrichtung 33 des dritten Roboters 31 als der Slave ausgebildet ist. Aufgrund einer Eingabe in ein mit der Steuervorrichtung 43 verbundenes Bediengerät 48 steuert die Steuervorrichtung 43 die elektrischen Antriebe des vierten Roboters 44 und gegebenenfalls der Lineareinheit 47 derart an, dass die Befestigungsvorrichtung 45 des vierten Roboters 44 und somit die Trägervorrichtung 41 eine Bewegung ausführen.

Gleichzeitig übermittelt die Steuervorrichtung 43 des vierten Roboters 44 der Steuervorrichtung 33 des dritten Roboters 31 eine Information über die aktuelle Position und Orientierung seiner Befestigungsvorrichtung 45, die im Falle des vorliegenden Ausführungsbeispiels eine Information über die Position und Orientierung eines dieser Befestigungsvorrichtung 45 zugeordneten Koordinatensystems darstellt. Der Steuervorrichtung 33 des dritten Roboters 31 steht außerdem eine Information über die relative Lage (Orientierung und Position) zwischen dem Koordinatensystem der Befestigungsvorrichtung 45 des dritten Roboters 44 und gegebenenfalls einem Koordinatensystem der Trägervorrichtung 41 und ein Koordinatensystem der Patientenliege 8 zur Verfügung.

Ferner wurden die Roboterarme 32, 42 des dritten und vierten Roboters 31, 44 vorab vermessen, sodass der Steuervorrichtung 33 des dritten Roboters 31 auch eine Information über die relative Lage der Roboterarme 32, 42 des dritten und des vierten Roboters 31, 44 zueinander bekannt ist. Eine Information über die aktuelle Position des mittels der Lineareinheit 46 verfahrbaren Roboterarms 42 des vierten Roboters 44 übermittelt die Steuervorrichtung 43 des vierten Roboters 44 ebenfalls der Steuervorrichtung 33 des dritten Roboters 31 während der Bewegung der Befestigungsvorrichtung 45 des vierten Roboters 44.

Somit ist es der Steuervorrichtung 33 des dritten Roboters 31 möglich, die aktuelle Position und Orientierung der Befestigungsvorrichtung 45 des vierten Roboters 44 bzw. der Trägervorrichtung 41 zu errechnen, um wiederum mittels eines auf der Steuervorrichtung 33 des dritten Roboters 31 laufenden Rechnerprogramms die Antriebe des dritten Roboters 31 derart anzusteuern, dass die Befestigungsvorrichtung 34 des dritten Roboters 31 und insbesondere ein Tool Center Point der Patientenliege 8 derart ausgerichtet wird, dass diese stets relativ zur Trägervorrichtung 41 gleichbleibend in vorgegebenen und errechneten Abstand ausgerichtet ist.

Die oben beschriebenen Informationen übermittelt die Steuervorrichtung 43 des vierten Roboters 44 kontinuierlich der Steuervorrichtung 33 des dritten Roboters 31 während der Bewegung, sodass die Steuervorrichtung 33 des dritten Roboters 31 stets die Antrieb des dritten Roboters 31 derart ansteuern kann, dass die Befestigungsvorrichtung 34 des dritten Roboters 31 und insbesondere der Tool Center Point der Patientenliege 8 derart ausgerichtet wird, dass diese stets relativ zur Trägervorrichtung 41 gleichbleibend ausgerichtet ist.

Im Falle des vorliegenden Ausführungsbeispiels kann die Röntgenvorrichtung 40 in einem zweiten Betriebsmodus betrieben werden, in dem z.B. der Arzt die Trägervorrichtung 41 manuell z.B. durch Führen, insbesondere durch Drücke oder Ziehen am Roboterarm 42 des vierten Roboters 44 oder an der Trägervorrichtung 41 manuell bewegt. Während des manuellen Führens übermittelt die Steuervorrichtung 43 des vierten Roboters 44 kontinuierlich eine Information über die Position und Orientierung des Koordinatensystems der Befestigungsvorrichtung 45 des vierten Roboters 44 sowie gegebenenfalls die Position des vierten Roboters 44 bezüglich der Lineareinheit 46.

Aufgrund dieser Information ist der Steuervorrichtung 33 des dritten Roboters 31 die Lage der Trägervorrichtung 41 während der manuellen Bewegung des vierten Roboters 44 stets bekannt. Demnach kann die Steuervorrichtung 33 des dritten Roboters 31 während der Bewegung stets den Antrieb des dritten Roboters 31 derart ansteuern, dass die Patientenliege 8 stets relativ Trägervorrichtung 41 gleichbleibend ausgerichtet ist.

## Patentansprüche

1. Medizinischer Arbeitsplatz, aufweisend
- ein medizintechnisches Gerät (1, 40), welches eine medizintechnische Einrichtung (RE, RQ, 41) und wenigstens einen ersten Roboter (21, 22, 44) aufweist, der einen mehrere Glieder aufweisenden ersten Roboterarm (23, 26, 42) und einen eine Bewegung des ersten Roboterarms (23, 26, 42) steuernde erste Steuervorrichtung (24, 27, 43) aufweist, wobei an einer ersten Befestigungsvorrichtung (25, 28, 45) des ersten Roboterarms (23, 26, 42) die medizintechnische Einrichtung (RE, RQ, 41) befestigt ist, und
- eine Patientenlagerungsvorrichtung (2), die eine Patientenliege (8) und einen zweiten Roboter (31) aufweist, der einen mehrere Glieder aufweisenden zweiten Roboterarm (32) und einen eine Bewegung des zweiten Roboterarms (32) steuernde zweite Steuervorrichtung (33) aufweist, wobei an einer zweiten Befestigungsvorrichtung (34) des zweiten Roboterarms (32) die Patientenliege (8) befestigt ist,
**gekennzeichnet dadurch, dass** die beiden Steuervorrichtungen (23, 26, 42, 33) miteinander gekoppelt und als ein Master-Slave-System ausgebildet sind, bei dem eine der Steuervorrichtungen (23, 42) als der Master und die andere Steuervorrichtung (33) als der Slave ausgebildet ist, die als Master ausgebildete Steuervorrichtung (23, 42) die als Slave ausgebildete Steuervorrichtung (33) derart ansteuert, dass bei einer ersten Bewegung der Befestigungsvorrichtung (25, 45) des Roboters (21, 44), dessen Steuervorrichtung (23, 42) als der Master ausgebildet ist, die als Slave ausgebildete Steuervorrichtung (33) ihren Roboterarm (32) derart bewegt, sodass die Befestigungsvorrichtung (34) des Roboters (31), dessen Steuervorrichtung (33) als Slave ausgebildet ist, eine zweite Bewegung ausführt, aufgrund derer die Patientenliege (8) und die medizintechnische Einrichtung (RE, RQ, 41) stets relativ zueinander gleichbleibend ausgerichtet sind.

2. Medizinischer Arbeitsplatz nach Anspruch 1, bei der die als Master ausgebildete Steuervorrichtung (23, 42) ihren Roboterarm (23, 26, 42) derart automatisch ansteuert, dass die relevante Befestigungsvorrichtung (25, 28, 45) die erste Bewegung durchführt.

3. Medizinischer Arbeitsplatz nach Anspruch 1, bei der die erste Bewegung aufgrund eines manuellen Führens zumindest eines Teils der medizintechnischen Einrichtung (RE, RQ, 41) oder aufgrund eines manuellen Führens der Patientenliege (8) erfolgt.

4. Medizinischer Arbeitsplatz nach Anspruch 1, aufweisend mit der als Master Steuervorrichtung (24, 43) gekoppelte Eingabemittel (29, 48), mittels deren die als Master ausgebildete Steuervorrichtung (24, 43) ihren Roboterarm (23, 42) derart ansteuert, dass die relevante Befestigungsvorrichtung (25, 45) die erste Bewegung aufgrund einer manuellen Eingabe in die Eingabemittel (29, 48) durchführt.

5. Medizinischer Arbeitsplatz nach einem der Ansprüche 1 bis 4, bei der
- die als Master ausgebildete Steuervorrichtung (24, 43) der als Slave ausgebildeten Steuervorrichtung (33) während der ersten Bewegung eine Information über die aktuelle Position und Orientierung ihrer Befestigungsvorrichtung (25, 45) übermittelt, und
- die als Slave ausgebildete Steuervorrichtung (33) aufgrund der relativen Lage des Roboterarm (23, 42), dessen Steuervorrichtung (24, 43) als Master ausgebildet ist, relativ zum Roboterarm (32), dessen Steuervorrichtung (33) als Slave ausgebildet ist, und der Information über die aktuelle Position und Orientierung der Befestigungsvorrichtung (25, 45) des Roboters (21, 44), dessen Steuervorrichtung (24, 43 als Master ausgebildet ist, den Roboterarm (32) des Roboters (31), dessen Steuervorrichtung (33) als Slave ausgebildet ist, derart bewegt, so dass die Befestigungsvorrichtung (34) dieses Roboters (31) eine Position und Orientierung aufweist, in der die Patientenliege (8) und die medizintechnische Einrichtung (RE, RQ, 41) stets relativ zueinander gleichbleiben ausgerichtet sind.

6. Medizinischer Arbeitsplatz nach einem der Ansprüche 1 bis 4, bei dem die als Master ausgebildete Steuervorrichtung (24, 43), die Teil des medizinischen Gerätes (1, 40) ist, eine Information über die aktuelle Position und Orientierung der an ihrer Befestigungsvorrichtung (25, 45) angeordneten medizintechnische Einrichtung (RE, RQ, 41) der Steuervorrichtung (33) übermittelt, die als Slave ausgebildet ist.

7. Medizinischer Arbeitsplatz nach einem der Ansprüche 1 bis 4, bei dem die als Master ausgebildete Steuervorrichtung Teil der Patientenlagerungsvorrichtung (2) ist, und diese Steuervorrichtung (33) eine Information über die aktuelle Position und Orientierung der an ihrer Befestigungsvorrichtung (36) angeordneten Patientenliege (8) übermittelt.

8. Medizinischer Arbeitsplatz nach einem der Ansprüche 1 bis 7, bei dem die medizintechnische Einrichtung (RE, RQ, 41) als eine bildgebende medizintechnische Einrichtung ausgebildet ist.

9. Medizinischer Arbeitsplatz nach Anspruch 8, bei dem die bildgebende medizintechnische Einrichtung (RE, RQ, 41) als eine Röntgenvorrichtung ausgebildet ist.

## Claims

1. Medical workstation, comprising
- a medical technology apparatus (1, 40), which comprises a medical technology device (RE, RQ, 41) and at least one first robot (21, 22, 44) that comprises a first robot arm (23, 26, 42) having a plurality of links and a first control device (24, 27, 43) that controls a movement of first robot arm (23, 26, 42), the medical technology device (RE, RQ, 41) being attached to a first fastening device (25, 28, 45) of the first robot arm (23, 26, 42), and
- a patient support device (2) that comprises a patient table (8) and a second robot (31), which has a second robot arm (32) with a plurality of links and a second control device (33) controlling a movement of the second robot arm (32), the patient table (8) being attached to a second fastening device (34) of the second robot arm (32),
**characterised in that**
- the two control devices (23, 26, 42, 33) are coupled to each other and designed as a master-slave system, wherein one of the control devices (23, 42) is designed as the master and the other control device (33) is designed as the slave, which control device (23, 42) designed as the master activates the control device (33) designed as the slave in such a way that during a first movement of the fastening device (25, 45) of the robot (21, 44), whose control device (23, 42) is designed as the master, the control device (33) designed as the slave moves its robot arm (32) in such a way that the fastening device (34) of the robot (31), whose control device (33) is designed as the slave, performs a second movement, on the basis of which the patient table (8) and the medical technology device (RE, RQ, 41) are always oriented in a constant manner relative to each other.

2. Medical workstation according to claim 1, wherein the control device (23, 42) designed as the master automatically activates its robot arm (23, 26, 42) in such a way that the relevant fastening device (25, 28, 45) performs the first movement.

3. Medical workstation according to claim 1, wherein the first movement is performed on the basis of the manual guiding of at least a part of the medical technology device (RE, RQ, 41) or on the basis of the manual guiding of the patient table(8).

4. Medical workstation according to claim 1, comprising input means (29, 48) that are coupled to the control device (24, 43) designed as the master, by means of which the control device (24, 43) designed as the master activates its robot arm (23, 42) in such a way that the relevant fastening device (25, 45) performs the first movement on the basis of manual input into the input means (29, 48).

5. Medical workstation according to any one of claims 1 to 4, wherein
- during the first movement the control device (24, 43) designed as the master transmits to the control device (33) designed as the slave information about the current position and orientation of its fastening device (25, 45), and
- on the basis of the relative position of the robot arm (23, 42), whose control device (24, 43) is designed as the master, relative to the robot arm (32), whose control device is designed as the slave (33), and on the basis of the information about the current position and orientation of the fastening device (25, 45) of the robot (21, 44), whose control device (24, 43) is designed as the master, the control device (33) designed as the slave moves the robot arm (32) of the robot (31), whose control device (33) is designed as the slave, in such a way that the fastening device (34) of said robot (31) has a position and orientation in which the orientations of the patient table (8) and the medical technology device (RE, RQ, 41) are always constant relative to one other.

6. Medical workstation according to any one of claims 1 to 4, wherein the control device (24, 43) designed as the master, which is part of the medical technology apparatus (1, 40), transmits information about the current position and orientation of the medical technology device (RE, RQ, 41) that is arranged on its fastening device (25, 45) to the control device (33) which is designed as the slave.

7. Medical workstation according to any one of claims 1 to 4, wherein the control device designed as the master is part of the patient support device (2), and said control device (33) transmits information about the current position and orientation of the patient table (8) arranged on its fastening device (36).

8. Medical workstation according to any one of claims 1 to 7, wherein the medical technology device (RE, RQ, 41) is designed as an imaging medical technology device.

9. Medical workstation according to claim 8, wherein the imaging medical technology device (RE, RQ, 41) is designed as an X-ray device.

## Revendications

1. Poste de travail médical, comportant
- un appareil médico-technique (1, 40) qui présente un dispositif médico-technique (RE, RQ, 41) et au moins un premier robot (21, 22, 44) qui présente un premier bras de robot (23, 26, 42) comportant plusieurs membres et un premier dispositif de commande (24, 27, 43) commandant un mouvement du premier bras de robot (23, 26, 42), le dispositif médico-technique (RE, RQ, 41) étant fixé sur un premier dispositif de fixation (25, 28, 45) du premier bras de robot (23, 26, 43), et
- un dispositif d'installation de patient (21) qui présente une table de patient (8) et un deuxième robot (31) qui présente un deuxième bras de robot (32) comportant plusieurs membres et un deuxième dispositif de commande (33) commandant un mouvement du deuxième bras de robot (32), la table de patient (8) étant fixée sur un deuxième dispositif de fixation (34) du deuxième bras de robot (32),
**caractérisé en ce que** les deux dispositifs de commande (23, 26, 42, 33) sont accouplés l'un à l'autre et réalisés sous forme de système maître-esclave, dans lequel un des dispositifs de commande (23, 42) est réalisé en tant que maître et l'autre dispositif de commande (33) en tant qu'esclave, le dispositif de commande (23, 42) réalisé en tant que maitre commande le dispositif de commande réalisé en tant qu'esclave (33) de telle sorte que lors d'un premier mouvement du dispositif de fixation (25, 45) du robot (21, 44), dont le dispositif de commande (23, 42) est réalisé en tant que maitre, le dispositif de commande (33) réalisé en tant qu'esclave déplace son bras de robot (32) de telle sorte que le dispositif de fixation (34) du robot (31), dont le dispositif de commande (33) est réalisé en tant qu'esclave, exécute un deuxième mouvement en raison duquel la table de patient (8) et le dispositif médico-technique (RE, RQ, 41) sont toujours orientés de manière constante l'un par rapport à l'autre.

2. Poste de travail médical selon la revendication 1, dans lequel le dispositif de commande (23, 42) réalisé en tant que maître pilote automatiquement son bras de robot (23, 26, 42) de telle sorte que le dispositif de fixation (25, 28, 45) concerné exécute le premier mouvement.

3. Poste de travail médical selon la revendication 1 dans lequel le premier mouvement a lieu en raison d'un guidage manuel d'au moins une partie du dispositif médico-technique (RE, RQ, 41) ou en raison d'un guidage manuel de la table de patient (8).

4. Poste de travail médical selon la revendication 1, présentant des moyens d'entrée (29, 48) accouplés au dispositif de commande (24, 43) réalisé en tant que maître, au moyen desquels le dispositif de commande (24, 43) réalisé en tant que maître pilote son bras de robot (23, 42) de telle sorte que le dispositif de fixation (25, 45) concerné exécute le premier mouvement suite à une introduction manuelle dans les moyens d'entrée (29, 48).

5. Poste de travail médical selon l'une des revendications 1 à 4, dans lequel
- le dispositif de commande (24, 43) réalisé en tant que maître transmet au dispositif de commande (33) réalisé en tant qu'esclave, pendant le premier mouvement, une information sur les position et orientation actuelles de son dispositif de fixation (25, 45), et
- le dispositif de commande (33) réalisé en tant qu'esclave, en raison de la position relative du bras de robot (23, 42) dont le dispositif de commande (24, 43) est réalisé en tant que maître, par rapport au bras de robot (32) dont le dispositif de commande (33) est réalisé en tant qu'esclave, et en raison de l'information sur les position et orientation actuelles du dispositif de fixation (25, 45) du robot (21, 44), dont le dispositif de commande (24, 43) est réalisé en tant que maître, déplace le bras de robot (32) du robot (31), dont le dispositif de commande (33) est réalisé en tant qu'esclave, de telle sorte que le dispositif de fixation (34) de ce robot (31) présente une position et une orientation dans laquelle la table de patient (8) et le dispositif médico-technique (RE, RQ, 41) sont toujours orientés de manière constante l'une par rapport à l'autre..

6. Poste de travail médical selon l'une des revendications 1 à 4, dans lequel dans lequel le dispositif de commande (24, 43) réalisé en tant que maître, qui fait partie de l'appareil médical (1, 40), transmet au dispositif de commande (33) qui est réalisé en tant qu'esclave une information sur les position et orientation actuelles du dispositif médico-technique (RE, RQ, 41) agencé sur son dispositif de fixation (25, 45).

7. Poste de travail médical selon l'une des revendications 1 à 4, dans lequel le dispositif de commande réalisé en tant que maître fait partie du dispositif d'installation de patient (2), et ce dispositif de commande (33) transmet une information sur les position et orientation actuelles de la table de patient (8) agencée sur son dispositif de fixation (36).

8. Poste de travail médical selon l'une des revendications 1 à 7, dans lequel le dispositif médico-technique (RE, RQ, 41) est réalisé sous forme d'un dispositif médico-technique d'imagerie.

9. Poste de travail médical selon la revendication 8, dans lequel le dispositif médico-technique d'imagerie (RE, RQ, 41) est réalisé en tant que dispositif de radiographie.
